# EUROPÄISCHE PATENTSCHRIFT

(11) **EP 3 218 711 B1**
(45) Veröffentlichungstag und Bekanntmachung des Hinweises auf die Patenterteilung: **19.06.2019**
(21) Anmeldenummer: 15794519.7
(22) Anmeldetag: 09.11.2015
(51) Int. Cl.: G01N 33/22, C01B 3/00, G01N 29/024, G01N 9/36, G01N 21/41, H01M 8/04082

(54) **VERFAHREN UND VORRICHTUNG ZUM MESSEN DES HYDRIERGRADES EINES FLÜSSIGEN ORGANISCHEN WASSERSTOFFTRÄGERS**
METHOD AND DEVICE FOR MEASURING THE HYDROGENATION LEVEL OF A LIQUID ORGANIC HYDROGEN CARRIER
PROCÉDÉ ET DISPOSITIF DE MESURE DU DEGRÉ D'HYDROGÉNATION D'UN PORTEUR D'HYDROGÈNE ORGANIQUE LIQUIDE

(30) Priorität: 10.11.2014 DE 102014116345
(43) Veröffentlichungstag der Anmeldung: 20.09.2017
(73) Patentinhaber: Friedrich-Alexander-Universität Erlangen-Nürnberg, 91054 Erlangen (DE); Hydrogenious Technologies GmbH, 91058 Erlangen (DE)
(72) Erfinder: ARLT, Prof. Wolfgang, 90425 Nürnberg (DE); STARK, Katharina, 96215 Lichtenfels (DE); WASSERSCHEID, Peter, 91054 Erlangen (DE)
(74) Vertreter: Patentship Patentanwaltsgesellschaft mbH
(86) Internationale Anmeldenummer: PCT/EP2015/076056
(87) Internationale Veröffentlichungsnummer: WO 2016/075077

(56) Entgegenhaltungen:
- US-A1- 2007 034 532
- US-A1- 2008 138 674
- Christian Papp ET AL: "Wasserstoff, chemisch gespeichert", NACHRICHTEN AUS DER CHEMIE, vol. 62, no. 10, 2 October 2014 (2014-10-02), pages 963-969, XP55461681, DE ISSN: 1439-9598, DOI: 10.1002/nadc.201490355

## Beschreibung

Die vorliegende Erfindung betrifft ein Verfahren und eine Messeinrichtung zum Erfassen eines Hydriergrades einer Flüssigkeit, die einen oder mehrere hydrierbare flüssige Wasserstoffträger umfasst.

Die Druckschrift EP 1 475 349 beschreibt organische Hydride als Wasserstoffspeicher.

Die Druckschrift EP 2 042 850 beschreibt, dass der Kohlenstoffgehalt eines Gemisches mittels einer Dichtemessung und mischungsabhängigen Größen bestimmt werden kann. Die Dichte eines Alkans nimmt mit dem Molgewicht gleich der Anzahl der Kohlenstoffatome zu. Um andere Stoffe als Alkane im Gemisch zu erfassen, wird eine von der Dichte verschiedene Mischungsgröße gemessen.

Die Druckschrift US 2007/034532 A1 beschreibt Systeme und Verfahren zum Verteilen eines Produkts, welches freisetzbar in einem Träger eingebracht ist, sowie Systeme und Verfahren, um das Produkt zu entfernen, bzw. dem Träger zu erneuern.

Allerdings ist in diesen Druckschriften nicht beschrieben, wie eine physikalische Stoffgröße eine Korrelation zu mehr als zwei Komponenten herstellt.

Es ist die der Erfindung zugrundeliegende Aufgabe, einen Hydriergrad eines Gemischs von flüssigen Wasserstoffträgern auf einfache Weise zu bestimmen.

Diese Aufgabe wird durch Gegenstände mit den Merkmalen nach den unabhängigen Ansprüchen gelöst. Vorteilhafte Ausführungsformen der Erfindung sind Gegenstand der Figuren, der Beschreibung und der abhängigen Ansprüche.

Gemäß einem ersten Aspekt der Erfindung wird die Aufgabe durch ein Verfahren zum Erfassen eines Hydriergrades einer Flüssigkeit gelöst, die einen oder mehrere hydrierbare flüssige Wasserstoffträger umfasst, mit den Schritten eines Erfassens einer Stoffeigenschaft der Flüssigkeit; und eines Bestimmens des Hydriergrades der Flüssigkeit auf Basis der erfassten Stoffeigenschaft der Flüssigkeit. Unter dem Hydriergrad wird der Grad der Sättigung der im Ausgangszustand des Wasserstoffträgers vorhandenen ungebundenen Elektronen durch chemische Bindung mit Wasserstoff verstanden. Bei einem nicht mit Wasserstoff beladenem Wasserstoffträger mit einer maximalen Zahl an ungebundenen Elektronen beträgt der Hydriergrad 0. Bei einem vollständig mit Wasserstoff beladenem Wasserstoffträger, bei dem alle Doppelbindungen durch chemische Bindung mit Wasserstoff gesättigt sind, und folglich eine minimale Zahl an ungebundenen Elektronen in dem Wasserstoffträger vorhanden ist, beträgt der Hydriergrad 1.

Dadurch wird beispielsweise der technische Vorteil erreicht, dass der Hydriergrad eines Vielkomponentensystems mithilfe nur einer thermophysikalischen Messgröße bestimmt werden kann. Bei einem Vielkomponentensystem, einer Mischung mit zumindest zwei Stoffen, ist es generell schwer mit nur einem experimentell ermittelten Parameter eine eindeutige Konzentrationsbestimmung der Stoffe in der Mischung durchzuführen. Überraschend konnte bei dem Vielkomponentensystem der vorliegenden Erfindung, welches eine Mischung von zumindest zwei Stoffen umfasst, eine eindeutige Funktion zwischen dem Hydriergrad der Flüssigkeit und einer Stoffeigenschaft, wie beispielsweise Dichte oder Brechungsindex, bestimmt werden. Dies gelingt insbesondere dadurch, dass die Stoffe eine identische chemische Grundstruktur aufweisen und keine spezifische Konzentrationsbestimmung der Stoffe in der Mischung durchgeführt wird, sondern dass für die Stoffe als eine einzige einheitliche Größe ausschließlich der Hydriergrad der Flüssigkeit bestimmt wird.

Beispielweise kann bei einem erfindungsgemäßen Vielkomponentensystem mit zwei Stoffen der Hydriergrad als eine lineare Funktion des Brechungsindex angegeben werden. Dadurch kann der Hydriergrad des Stoffgemisches mithilfe nur einer thermophysikalischen Messgröße eindeutig bestimmt werden, wodurch eine rasche und effektive Erfassung des Hydriergrads von einem oder mehreren Wasserstoffträgern ermöglicht wird.

In einer vorteilhaften Ausführungsform des Verfahrens wird im Schritt des Erfassens der Stoffeigenschaft zumindest eine der folgenden Stoffeigenschaften erfasst: Dichte, optischer Brechungsindex, relative Permittivität, Schallgeschwindigkeit, Viskosität, Adsorption, Absorption oder zumindest eine Stoffeigenschaft, welche aus Stoffdaten wie Dichte der Flüssigkeit ableitbar ist.

Dadurch wird beispielsweise der technische Vorteil erreicht, dass die genannten Stoffeigenschaften der Flüssigkeit eine vorteilhafte Bestimmung des Hydriergrads des Vielkomponentensystems ermöglichen, da ein Zusammenhang, insbesondere ein linearer, ein nicht-linearer, z.B. ein exponentieller oder polynominaler, oder ein logarithmischer Zusammenhang zwischen den genannten Stoffeigenschaften und dem Hydriergrad des Stoffgemisches besteht. Unter der Stoffeigenschaft der Adsorption der Flüssigkeit wird die Adsorptionsfähigkeit der Flüssigkeit, oder die Adsorptionsfähigkeit von einem oder mehreren hydrierbaren flüssigen Wasserstoffträgern der Flüssigkeit, an eine Festkörperoberfläche bezeichnet. Unter der Stoffeigenschaft der Absorption der Flüssigkeit wird die Absorptionsfähigkeit der Flüssigkeit, oder die Absorptionsfähigkeit von einem oder mehreren hydrierbaren flüssigen Wasserstoffträgern der Flüssigkeit, in das Innere eines Festkörpers oder einer Flüssigkeit bezeichnet.

In einer vorteilhaften Ausführungsform des Verfahrens ist die Stoffeigenschaft ein optischer Brechungsindex, wobei der optische Brechungsindex mittels eines Refraktometers, eines Goniometers oder eines Michelson-Interferometers bestimmt wird.

Dadurch wird beispielsweise der technische Vorteil erreicht, dass eine präzise Erfassung des Brechungsindex erfolgt, die durch die Verwendung eines Refraktometers, eines Goniometers oder eines Michelson-Interferometers rasch durchgeführt werden kann, und gegebenenfalls auch automatisch ablaufen kann. Die Bestimmung des Brechungsindex mittels Refraktometer, Goniometer oder Michelson-Interferometers ist zudem mit einer hohen Genauigkeit möglich, wodurch eine vorteilhafte Bestimmung des Hydriergrades der Wasserstoffträger gewährleistet wird.

In einer weiteren vorteilhaften Ausführungsform des Verfahrens umfasst das Verfahren die Schritte eines Erfassens der Dichte der Flüssigkeit und eines Bestimmens des Hydriergrades auf Basis der Dichte der Flüssigkeit.

Dadurch wird beispielsweise der technische Vorteil erreicht, dass die Genauigkeit des Verfahrens durch das zusätzliche Erfassen der Dichte der Flüssigkeit erhöht wird. Wenn zusätzlich zu der erfassten Stoffeigenschaft der Flüssigkeit noch die Dichte der Flüssigkeit erfasst wird, stehen zwei experimentell bestimmte Parameter zur Verfügung, um den Hydriergrad des erfindungsgemäßen Vielkomponentengemisches zu bestimmen. Dadurch kann die Genauigkeit der Erfassung gesteigert werden und der Hydriergrad der Wasserstoffträger kann vorteilhaft bestimmt werden.

Erfindungsgemäß umfasst die Flüssigkeit eine Mischung aus Dibenzyltoluol und zumindest teilweise hydrierten Dibenzyltoluol, oder eine Mischung aus Benzyltoluol und zumindest teilweise hydrierten Benzyltoluol, oder eine Mischung aus Benzyltoluol, Dibenzyltoluol, zumindest teilweise hydrierten Dibenzyltoluol und zumindest teilweise hydrierten Benzyltoluol.

Dadurch wird beispielsweise der technische Vorteil erreicht, dass durch die genannten Verbindungen besonders effiziente Wasserstoffträger ausgewählt werden. Bei ungesättigten zyklischen Kohlenwasserstoffverbindungen steht eine Vielzahl von ungesättigten Kohlenstoff-Kohlenstoff-Doppelbindungen zur Verfügung, die mit Wasserstoffmolekülen unter Bildung von gesättigten Kohlenstoff-Kohlenstoff-Doppelbindungen reagieren können, wodurch Wasserstoff gespeichert werden kann. Beispielsweise verfügt ein ((5-Methyl-1,3-phenylen)bis(methylen)dibenzol Molekül über neun ungesättigte Kohlenstoff-Kohlenstoff-Doppelbindungen, die neun Wasserstoffmoleküle im Rahmen einer vollständigen Hydrierung durch Wasserstoff aufnehmen können. Durch das hohe Verhältnis von gespeicherten Wasserstoffmolekülen pro zyklischer Kohlenwasserstoffverbindung kann eine wirksame Wasserstoffspeicherung in einem begrenzten Volumen von Flüssigkeit ermöglicht werden.

Eine ungesättigte zyklische Kohlenwasserstoffverbindung umfasst jede Kohlenwasserstoffverbindung, welche zumindest einen Kohlenwasserstoffring umfasst, und welche zumindest eine Kohlenstoff-Kohlenstoff-Doppelbindung umfasst. Die ungesättigte zyklische Kohlenwasserstoffverbindung kann eine aromatische oder nicht aromatische zyklische Kohlenwasserstoffverbindung umfassen. Die ungesättigte zyklische Kohlenwasserstoffverbindung kann eine Kohlenwasserstoffverbindung mit einem oder mehreren Kohlenwasserstoffringen umfassen, wobei bei einer Vielzahl von Kohlenwasserstoffringen, die Kohlenwasserstoffringe anellierte Kohlenwasserstoffringe oder durch Substituenten verbundene Kohlenwasserstoffringe umfassen können.

In der ungesättigten zyklischen Kohlenwasserstoffverbindung kann zumindest ein Kohlenstoffatom durch ein Heteroatom umfassend Sauerstoff, Schwefel oder Stickstoff, ersetzt sein. Jedes Kohlenstoff- oder Heteroatom der ungesättigten zyklischen Kohlenwasserstoffverbindung kann mit einem Substituent substituiert sein, der ausgewählt ist aus der Gruppe bestehend aus Methyl, Ethyl, Propyl, Butyl, Chlor, Fluor oder Brom.

Eine zumindest teilweise hydrierte ungesättigte zyklische Kohlenwasserstoffverbindung umfasst eine zyklische Kohlenwasserstoffverbindung, bei der zumindest eine Kohlenstoff-Kohlenstoff-Doppelbindung der zyklischen Kohlenwasserstoffverbindung durch eine Kohlenstoff-Kohlenstoff-Einfachbindung ersetzt ist. Bevorzugt umfasst eine zumindest teilweise hydrierte ungesättigte zyklische Kohlenwasserstoffverbindung eine vollständig hydrierte zyklische Kohlenwasserstoffverbindung, bei der alle Kohlenstoff-Kohlenstoff-Doppelbindungen der zyklischen Kohlenwasserstoffverbindung durch Kohlenstoff-Kohlenstoff-Einfachbindungen ersetzt sind.

Die Mischung aus einer ungesättigten zyklischen Kohlenwasserstoffverbindung und einer zumindest teilweise hydrierten ungesättigten zyklischen Kohlenwasserstoffverbindung, kann verschiedene teilweise hydrierte ungesättigte zyklische Kohlenwasserstoffverbindungen mit verschiedenen Hydriergraden der ungesättigten zyklischen Kohlenwasserstoffverbindungen umfassen.

Der Begriff Benzyltoluol und Dibenzyltoluol umfasst zudem bevorzugt jedes Konstitutionsisomer und jedes Stereoisomer der genannten Verbindungen.

Ein zumindest teilweise hydriertes Dibenzyltoluol umfasst jede Dibenzyltoluol Verbindung, bei der zumindest eine Kohlenstoff-Kohlenstoff-Doppelbindung des Dibenzyltoluols durch eine Kohlenstoff-Kohlenstoff-Einfachbindung ersetzt ist, und bevorzugt alle Kohlenstoff-Kohlenstoff-Doppelbindungen durch Kohlenstoff-Kohlenstoff-Einfachbindungen ersetzt sind.

Ein zumindest teilweise hydriertes Benzyltoluol umfasst jede Benzyltoluol Verbindung, bei der zumindest eine Kohlenstoff-Kohlenstoff-Doppelbindung des Benzyltoluols durch eine Kohlenstoff-Kohlenstoff-Einfachbindung ersetzt ist, und bevorzugt alle Kohlenstoff-Kohlenstoff-Doppelbindungen durch Kohlenstoff-Kohlenstoff-Einfachbindungen ersetzt sind.

Bevorzugt umfasst ein zumindest teilweise hydriertes Dibenzyltoluol, 1-Benzyl-3-(cyclohexylmethyl)-5-methylbenzol, ((5-Methyl-1,3-phenylen)bis(methylen)dicyclohexan, ((5-Methylcyclohexan-1,3-diyl)bis(methylen)dicyclohexan, ((5-Methyl-1,3-phenylen)bis(methylen)dibenzol, 1-Benzyl-4-(cyclohexylmethyl)-2-methylbenzol, ((2-Methyl-1,4-phenylen)bis(methylen)dicyclohexan, ((2-Methylcyclohexan-1,4-diyl)bis(methylen)dicyclohexan, ((2-Methyl-1,4-phenylen)bis(methylen)dibenzol, 2-Benzyl-4-(cyclohexylmethyl)-1-methylbenzol, ((4-Methyl-1,3-phenylen)bis(methylen)dicyclohexan, ((4-Methylcyclohexan-1,3-diyl)bis(methylen)dicyclohexan, ((4-Methyl-1,3-phenylen)bis(methylen)dibenzol, 1-benzyl-3-(cyclohexylmethyl)-2-methylbenzol, ((2-methyl-1,3-phenylen)bis(methylen)dicyclohexan, ((2-methylcyclohexan-1,3-diyl)bis(methylen)dicyclohexan, ((2-Methyl-1,3-phenylen)bis(methylen)dibenzol, 1-Benzyl-2-(cyclohexylmethyl)-4-methylbenzol, ((4-Methyl-1,2-phenylen)bis(methylen)dicyclohexan, ((4-Methylcyclohexan-1,2-diyl)bis(methylen)dicyclohexan, oder ((4-Methyl-1,2-phenylen)bis(methylen)dibenzol.

Erfindungsgemäß wird der Hydriergrad der Flüssigkeit auf Basis eines linearen Zusammenhangs des Hydriergrades und der erfassten Stoffeigenschaft bestimmt.

Dadurch wird beispielsweise der technische Vorteil erreicht, dass der Hydriergrad auf einfache und schnelle Weise berechnet werden kann, und dadurch eine effiziente und automatisierte Bestimmung des Hydriergrades der Flüssigkeit, beispielsweise in einem Tank, erreicht werden kann.

In einer vorteilhaften Ausführungsform des Verfahrens wird die Stoffeigenschaft, insbesondere Dichte, mittels eines Aräometers, eines Pyknometers, einer hydrostatischen Wägung oder einer Schwingungsmessung bestimmt. Dadurch wird beispielsweise der technische Vorteil erreicht, dass eine genaue Erfassung der Stoffeigenschaft, insbesondere Dichte, erfolgt. Damit ist ferner eine Messung des Hydriergrads der Flüssigkeit im Labor als auch in technischen Ausführungsformen möglich, wobei die Stoffeigenschaft der Flüssigkeit, insbesondere Dichte, mit einer hohen Präzision bestimmt werden kann.

Gemäß einem zweiten Aspekt der Erfindung wird die Aufgabe durch eine Messeinrichtung zum Erfassen eines Hydriergrades einer Flüssigkeit gelöst, die einen oder mehrere hydrierbare flüssige Wasserstoffträger umfasst, mit einer Erfassungseinrichtung zum Erfassen einer Stoffeigenschaft der Flüssigkeit; und einer Bestimmungseinrichtung zum Bestimmen des Hydriergrades auf Basis der erfassten Stoffeigenschaft der Flüssigkeit. Dadurch wird beispielsweise ebenfalls der technische Vorteil erreicht, dass der Hydriergrad eines Vielkomponentensystems mithilfe nur einer thermophysikalischen Messgröße bestimmen werden kann.

In einer vorteilhaften Ausführungsform der Messeinrichtung ist die Erfassungseinrichtung ausgebildet, zumindest eine der folgenden Stoffeigenschaften zu erfassen: Dichte, optischer Brechungsindex, relative Permittivität, Schallgeschwindigkeit, Viskosität, Adsorption, Absorption oder zumindest eine Stoffeigenschaft, welche aus Stoffdaten wie Dichte der Flüssigkeit ableitbar ist.

Dadurch wird beispielsweise der technische Vorteil erreicht, dass die genannten Stoffeigenschaften der Flüssigkeit eine vorteilhafte Bestimmung des Hydrierungsgrads des Vielkomponentensystems ermöglichen.

In einer vorteilhaften Ausführungsform der Messeinrichtung ist die Stoffeigenschaft ein optischer Brechungsindex, und wobei die Erfassungseinrichtung ein Refraktometer, ein Goniometer oder ein Michelson-Interferometer umfasst. Dadurch wird beispielsweise der technische Vorteil erreicht, dass der Brechungsindex mit einer hohen Präzision ermittelt werden kann.

In einer weiteren vorteilhaften Ausführungsform der Messeinrichtung umfasst die Messeinrichtung eine Erfassungseinrichtung zum Erfassen von Daten der Flüssigkeit in einem Tank, insbesondere Gewicht oder Volumen. Dadurch wird beispielsweise der technische Vorteil erreicht, dass die Menge der Flüssigkeit für weitere Berechnungen herangezogen werden kann. Dadurch kann eine effiziente und genaue Bestimmung des Hydriergrads der Flüssigkeit in dem Tank durchgeführt werden.

Erfindungsgemäß umfasst die Messeinrichtung eine Berechnungseinrichtung zum Berechnen einer gespeicherten oder speicherbaren Energiemenge in dem Tank auf Basis des Hydriergrades und der Menge der Flüssigkeit. Dadurch wird beispielsweise der technische Vorteil erreicht, dass der Energieinhalt des Tanks einem Benutzer angezeigt werden kann, wodurch der Benutzer den verbleibenden Energieinhalt der Flüssigkeit in dem Tank besser einschätzen kann.

In einer vorteilhaften Ausführungsform der Messeinrichtung umfasst die Messeinrichtung eine Tankuhr zum Anzeigen der gespeicherten oder speicherbaren Energiemenge in dem Tank. Dadurch wird beispielsweise der technische Vorteil erreicht, dass durch die Tankuhr bei einem bekannten Energieverbrauch des Tanks in einem bestimmten Zeitintervall, wie z.B. bei einem konstanten Wasserstoffverbrauch eines Tanks eines Fahrzeugs, durch die Tankuhr eine Prognose und Anzeige der Restenergiemenge, bzw. Restwasserstoffmenge, des Tanks möglich ist. So kann beispielweise bei einem Fahrzeug, welches Wasserstoff verbraucht, durch die Tankuhr die Restenergiemenge des Tanks, bzw. die Restreichweite des Automobils ermittelt und angezeigt werden.

In einer vorteilhaften Ausführungsform der Messeinrichtung umfasst die Erfassungseinrichtung ein Aräometer, ein Pyknometer, eine hydrostatische Waage oder eine Einrichtung zur Schwingungsmessung, insbesondere einen Biegeschwinger. Dadurch wird beispielsweise der technische Vorteil erreicht, dass die Dichte mit einer hohen Präzision bestimmt werden kann.

Gemäß einem dritten Aspekt der Erfindung wird die Aufgabe durch ein Fahrzeug mit einem Tank für eine Flüssigkeit gelöst, die einen oder mehrere hydrierbare flüssige Wasserstoffträger umfasst, mit der Messeinrichtung nach dem zweiten Aspekt. Dadurch wird beispielsweise der technische Vorteil erreicht, dass die Messeinrichtung für eine Tankuhr für das Fahrzeug verwendet werden kann.

Ausführungsbeispiele der Erfindung sind in den Zeichnungen dargestellt und werden im Folgenden näher beschrieben.

Es zeigen:
- Fig. 1: teilhydrierte Isomere von N-Ethylcarbazol;
- Fig. 2: Isomere von Dibenzyltoluol; und
- Fig. 3: eine schematische Ansicht einer Messeinrichtung zum Erfassen eines Hydriergrades einer Flüssigkeit.

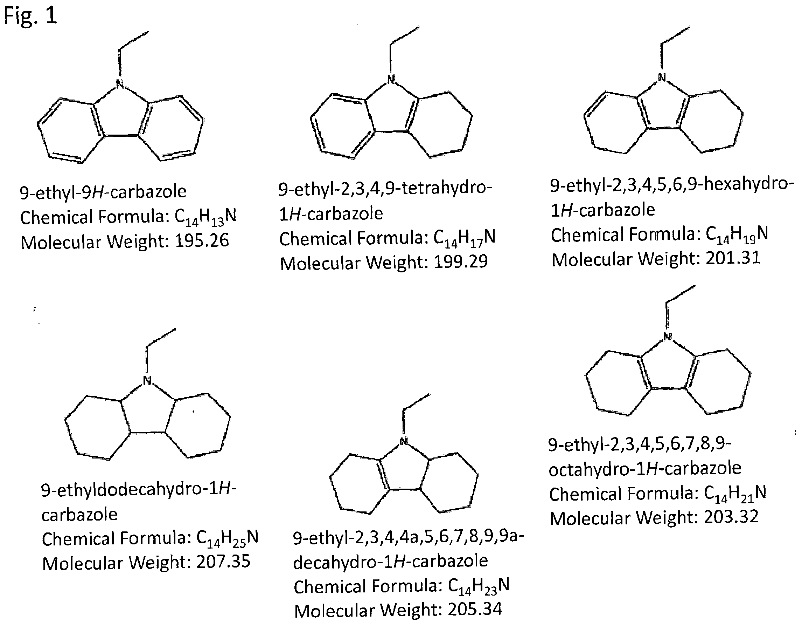

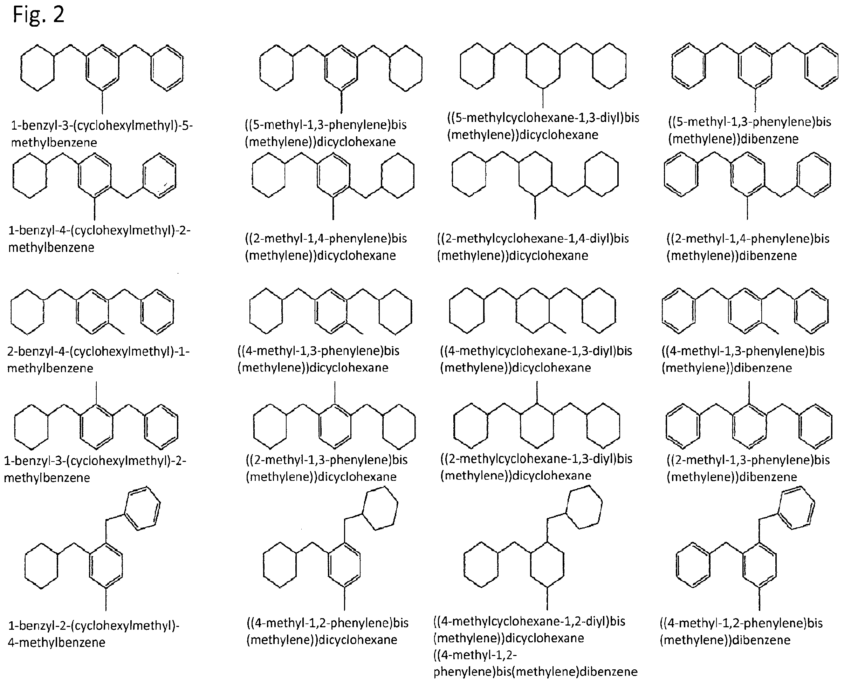

Fig. 1 zeigt teilhydrierte Isomere von N-Ethylcarbazol und Fig. 2 zeigt mögliche Isomere von Dibenzyltoluol. Beide Substanzen sind Beispiele für einen flüssigen Wasserstoffträger (LOHC - Liquid Organic Hydrogen Carrier), der aromatische Verbindungen umfasst.

Eine energiearme Form des flüssigen Wasserstoffträgers wird mittels katalysierter Hydrierung durch Wasserstoff reversibel in eine energiereiche Form gewandelt. In einer beispielsweise anders katalysierten Rückreaktion durch Temperaturerhöhung und/oder Reduzierung des Wasserstoffdruckes wird Wasserstoff wieder aus der hydrierten energiereichen Form unter Bildung der energiearmen Form zurückgewonnen. Die Reaktion ist reversibel, so dass der flüssige Wasserstoffträger von einem energiearmen zu einem energiereichen Ort im Kreis geführt werden kann, ohne sich selbst zu verbrauchen. Der flüssige Wasserstoffträger ist Transporteur für Energie in Form von Wasserstoff.

Besonders vorteilhaft einsetzbare flüssige Wasserstoffträger erlauben diese reversible Wandlung unter technisch relevanten Bedingungen, wie beispielsweise bei Drücken und Temperaturen, die technisch einfach darstellbar sind. Die Wasserstoffspeicherung in flüssigen Wasserstoffträgern weist den Vorteil auf, dass die flüssigen Wasserstoffträger unter den verwendeten Prozessbedingungen flüssig sind, drucklos in der Lagerung sind, und in ihren physikochemischen Eigenschaften hohe Ähnlichkeit zu herkömmlichen flüssigen Kraftstoffen aufweisen. Daher können Pumpen zum Transport und Behälter zur Lagerung aus dem Bereich der Kraftstoff- und Brennstofflogistik verwendet werden. Die Wasserstoffspeicherung in chemisch gebundener Form in einer organischen Flüssigkeit erlaubt eine drucklose Lagerung bei Normalbedingungen über große Zeiträume ohne signifikanten Wasserstoffverlust. Flüssige Wasserstoffträger basieren beispielhaft auf den Stoffen N-Ethylcarbazol, Benzyltoluol oder Dibenzyltoluol.

Aufgrund der unterschiedlichen Isomere können mehr als zwei Stoffe während des Hydrier- oder Dehydriervorgangs entstehen und zeitgleich in einer komplexen Mischung vorliegen. Die Flüssigkeit, die durch den flüssigen Wasserstoffträger gebildet wird, umfasst daher eine Anzahl von teil-, voll- oder unhydrierten Komponenten in unterschiedlicher Konzentration.

Da sich ein flüssiger Wasserstoffträger bei der Verwendung als Wasserstoff-Energielieferant nicht aufbraucht, wird die durch den Wasserstoffträger gebildete Flüssigkeit nicht aus einem Tank entfernt, sondern ändert nur ihren Energieinhalt aufgrund der wechselnden Konzentrationen der einzelnen Komponenten. Daher befindet sich im Tank stets ein Stoffpaar aus einer wasserstoffbeladenen und wasserstoffentladenen Form des flüssigen Wasserstoffträgers.

Eine Bestimmung des Hydriergrades h in diesem Vielkomponentengemisch könnte dadurch erfolgen, dass die jeweiligen Einzelkonzentrationen vermessen werden. Dieses Verfahren ist jedoch aufwändig. In einfacherer Weise lassen sich die thermophysikalischen Größen wie beispielsweise Dichte ρ und Brechungsindex n der Flüssigkeit zum Bestimmen eines Hydriergrades h verwenden.

Der Brechungsindex n ist eine dimensionslose physikalische Größe. Der Brechungsindex n gibt das Verhältnis der Vakuumlichtgeschwindigkeit zur Ausbreitungsgeschwindigkeit des Lichts in der Flüssigkeit an. Damit ist der Brechungsindex im Vakuum gleich 1 und steigt mit zunehmender Dichte an. Bei der Angabe sollte die Wellenlänge des Messlichtes notiert werden. Die Dichte ρ der Flüssigkeit ist die Masse der Flüssigkeit geteilt durch ihr Volumen, beispielsweise in Kilogramm pro Kubikmeter. Im Allgemeinen sind diese Größen miteinander korreliert.

Die Permittivität oder dielektrische Leitfähigkeit beschreibt die Durchlässigkeit eines Stoffes oder Stoffmischung für elektrische Felder. Die relative Permittivität εᵣ ist als das dimensionslose Verhältnis der Permittivität in Materie und im Vakuum definiert. Letztere wird als Feldkonstante bezeichnet. Anstelle der Feldkonstante wird oft auch die Permittivität von Luft als Referenzgröße verwendet.

Die relative Permittivität eines Stoffes oder Stoffmischung, beispielsweise einer Flüssigkeit, kann mithilfe eines Kondensators bestimmt werden. Mit einem LCR-Meter wird die Kapazität des Kondensators, gefüllt mit dem Stoff oder der Stoffmischung mit unbekannter Permittivität, bestimmt. Als Referenz dient die Kapazität desselben Kondensators gefüllt mit einer Substanz bekannter Permittivität, in der Regel Luft. Das Verhältnis der beiden Kapazitäten wird als relative Permittivität bezeichnet.

Die dynamische Viskosität ist ein Maß für die Zähigkeit einer Flüssigkeit oder eines Gases. Die Fließfähigkeit eines Stoffes nimmt mit steigender dynamischer Viskosität ab.

Die Verwendung des Brechungsindex n und/oder der Dichte ρ zur Bestimmung des Hydriergrades h ist insbesondere bei flüssigen Wasserstoffträgern vorteilhaft, wenn unhydrierte, teilhydrierte und vollhydrierte Formen des Wasserstoffträgers in einer Mischung zusammen in einem Tank gespeichert werden.

Bei einem Vielkomponentengemisch, welches aus Stoffen mit gleicher Grundstruktur besteht, ist der Hydriergrad eine lineare Funktion des Brechungsindex n und/oder der Dichte p. Dadurch kann eine einfache und effiziente Bestimmung des Hydriergrads der wasserstoffspeichernden Flüssigkeit anhand einer Messung des Brechungsindex n und/oder der Dichte ρ gewährleistet werden.

In einer weiteren Ausführungsform kann der Hydriergrad eine nicht-lineare oder eine logarithmische Funktion des Brechungsindex n und/oder der Dichte ρ sein. Ein nicht-linearer Zusammenhang kann beispielsweise einen exponentiellen oder einen polynominalen Zusammenhang umfassen. Dadurch dass der Hydriergrad eine nicht-lineare oder eine logarithmische Funktion des Brechungsindex n und/oder der Dichte ρ sein kann, wird eine einfache und effiziente Bestimmung des Hydriergrads ermöglicht. Durch die vorteilhafte Berechnung des Hydriergrads kann eine automatisierte Bestimmung des Hydriergrades der Flüssigkeit, beispielsweise in einem Tank, erreicht werden.

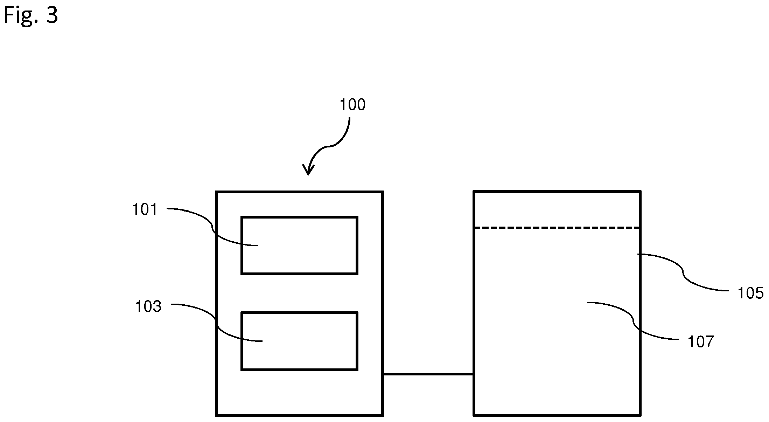

Fig. 3 zeigt eine schematische Ansicht einer Messeinrichtung 100 zum Erfassen eines Hydriergrades. Die Messeinrichtung 100 umfasst eine Erfassungseinrichtung 101 zum Erfassen einer Stoffeigenschaft der Flüssigkeit 107, wie beispielsweise den optischen Brechungsindex der Flüssigkeit 107 oder eine Dichte-Erfassungseinrichtung 101 zum Erfassen der Dichte der Flüssigkeit 107. Weiter umfasst die Erfassungseinrichtung 100 eine Bestimmungseinrichtung 103 zum Bestimmen des Hydriergrades auf Basis der erfassten Stoffeigenschaft der Flüssigkeit 107, wie z.B. dem optischen Brechungsindex oder der Dichte der Flüssigkeit 107. Beide thermophysikalischen Messgrößen sind auf einfache Weise zu messen. Die Bestimmungseinrichtung 103 kann beispielsweise durch einen Prozessor mit einem Speicher gebildet sein, der die Ausführung mathematischer Operationen zulässt.

Die Messeinrichtung 100 kann dabei als Tankuhr dienen, die anzeigt, wie hoch der Hydriergrad der Flüssigkeit 107 in dem Tank 105 ist. Die Tankuhr kann am Tank 105 selbst angebracht sein oder in Zu- und/oder Ableitungen zum Tank 105 eingebaut sein. Der Tankinhalt kann zur besseren Vermischung durch diese Leitungen umgepumpt werden. Die Messeinrichtung 100 kann in stationären oder mobilen Tanks 105 für die flüssigen Wasserstoffträger verwendet werden. Die im Tank 105 befindliche Menge eines flüssigen Wasserstoffträgers in Kilogramm ist beispielsweise durch Wägung beim Einfüllen bekannt.

Im stationären Fall kann die Messeinrichtung 100 anzeigen, wieviel Energie in Form von freisetzbarem Wasserstoff in dem Tank 105 enthalten ist und den Energieinhalt des Tanks 105 anzeigen. Daraus können Rückschlüsse auf eine zeitliche Reichweite des Tanks 105 gewonnen werden. Ein Benutzer des Tanks 105 für flüssige Wasserstoffträger kann auf diese Weise ermitteln, welche Energiemenge in dem Tank 105 speicherbar ist.

Die Messeinrichtung 105 kann außerdem zur Verfolgung der Hydrierung eines flüssigen Wasserstoffträgers in einem Reaktor eingesetzt werden, so dass ein chemischer Umsatz bestimmt werden kann. Die Messeinrichtung 100 kann zur Überwachung des Reaktionsfortschrittes bei chemischen Reaktoren verwendet werden, die Wasserstoff im flüssigen Wasserstoffträger speichern oder aus dem Wasserstoffträger Wasserstoff generieren.

Die Messeinrichtung 100 kann zur Anzeige der Reichweite eines Fahrzeugs verwendet werden, das einen Tank 105 für flüssige Wasserstoffträger umfasst, wenn der Energiebedarf pro km Fahrleistung bekannt ist. Die Reichweite ist direkt mit dem Energiegehalt verknüpft, der auf der Menge des flüssigen Wasserstoffträgers und dem Hydriergrad h basiert.

Das Fahrzeug verwendet den freigesetzten Wasserstoff zum Antrieb. Dazu ist eine Veränderung der energiereichen Form des flüssigen Wasserstoffträgers in die energiearme Form unter Bildung von Wasserstoff vorgesehen. Wenn das Fahrzeug einen einzigen Tank 105 für den flüssigen Wasserstoffträger besitzt, liegt in diesem Tank 105 eine Mischung hydrierter flüssiger Wasserstoffträger vor. Die Messeinrichtung 100 kann durch Differenzbildung zur Abrechnung des Energiebezugs an einer Tankstelle dienen, wenn ein teil- oder unhydrierter flüssiger Wasserstoffträger an die Tankstelle abgegeben wird und anschließend ein teil- oder vollhydrierter flüssiger Wasserstoffträger von der Tankstelle bezogen wird.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Der Schutzbereich der vorliegenden Erfindung ist durch die Ansprüche gegeben und wird durch die in der Beschreibung erläuterten oder den Figuren gezeigten Merkmale nicht beschränkt.

## Patentansprüche

1. Verfahren zum Erfassen eines Hydriergrades einer Flüssigkeit (107), die einen oder mehrere hydrierbare flüssige Wasserstoffträger umfasst, mit den Schritten:
Erfassen (S101) einer Stoffeigenschaft der Flüssigkeit (107); und
Bestimmen (S102) des Hydriergrades der Flüssigkeit (107) auf Basis der erfassten Stoffeigenschaft der Flüssigkeit (107),
**dadurch gekennzeichnet, dass** der Hydriergrad der Flüssigkeit (107) auf Basis eines linearen Zusammenhangs des Hydriergrades und der erfassten Stoffeigenschaft bestimmt wird, und
wobei die Flüssigkeit (107) eine Mischung aus Dibenzyltoluol und zumindest teilweise hydrierten Dibenzyltoluol, oder eine Mischung aus Benzyltoluol und zumindest teilweise hydrierten Benzyltoluol, oder eine Mischung aus Benzyltoluol, Dibenzyltoluol, zumindest teilweise hydrierten Dibenzyltoluol, und zumindest teilweise hydrierten Benzyltoluol umfasst.

2. Verfahren nach Anspruch 1, wobei im Schritt des Erfassens (S101) der Stoffeigenschaft zumindest eine der folgenden Stoffeigenschaften erfasst wird: Dichte, optischer Brechungsindex, relative Permittivität, Schallgeschwindigkeit, Viskosität, Adsorption, Absorption oder zumindest eine Stoffeigenschaft, welche aus Stoffdaten wie Dichte der Flüssigkeit (107) ableitbar ist.

3. Verfahren nach Anspruch 1 oder 2, wobei die Stoffeigenschaft ein optischer Brechungsindex ist, und wobei der optische Brechungsindex mittels eines Refraktometers, eines Goniometers oder eines Michelson-Interferometers bestimmt wird.

4. Verfahren nach einem der vorangehenden Ansprüche, weiter mit den Schritten eines Erfassens der Dichte der Flüssigkeit (107) und eines Bestimmens des Hydriergrades auf Basis der Dichte der Flüssigkeit (107).

5. Verfahren nach einem der vorstehenden Ansprüche, wobei die Stoffeigenschaft, insbesondere Dichte, mittels eines Aräometers, eines Pyknometers, einer hydrostatischen Wägung oder einer Schwingungsmessung bestimmt wird.

6. Messeinrichtung (100) zum Erfassen eines Hydriergrades einer Flüssigkeit (107), die einen oder mehrere hydrierbare flüssige Wasserstoffträger umfasst, mit:
einer Erfassungseinrichtung (101) zum Erfassen einer Stoffeigenschaft der Flüssigkeit (107); und
einer Bestimmungseinrichtung (103) zum Bestimmen des Hydriergrades auf Basis der erfassten Stoffeigenschaft der Flüssigkeit (107),
wobei der Hydriergrad der Flüssigkeit (107) auf Basis eines linearen Zusammenhangs des Hydriergrades und der erfassten Stoffeigenschaft bestimmt wird,
**dadurch gekennzeichnet, dass** die Messeinrichtung (100) eine Berechnungseinrichtung eingerichtet zum Berechnen einer gespeicherten oder speicherbaren Energiemenge in dem Tank (105) auf Basis des Hydriergrades und der Menge der Flüssigkeit (107) umfasst, wobei die Flüssigkeit (107) eine Mischung aus Dibenzyltoluol und zumindest teilweise hydrierten Dibenzyltoluol, oder eine Mischung aus Benzyltoluol und zumindest teilweise hydrierten Benzyltoluol, oder eine Mischung aus Benzyltoluol, Dibenzyltoluol, zumindest teilweise hydrierten Dibenzyltoluol, und zumindest teilweise hydrierten Benzyltoluol umfasst.

7. Messeinrichtung (100) nach Anspruch 6, wobei die Erfassungseinrichtung (101) ausgebildet ist, zumindest eine der folgenden Stoffeigenschaften zu erfassen: Dichte, optischer Brechungsindex, relative Permittivität, Schallgeschwindigkeit, Viskosität, Adsorption, Absorption oder zumindest eine Stoffeigenschaft, welche aus Stoffdaten wie Dichte der Flüssigkeit (107) ableitbar ist.

8. Messeinrichtung (100) nach Anspruch 6 oder 7, wobei die Stoffeigenschaft ein optischer Brechungsindex ist, und wobei die Erfassungseinrichtung (101) ein Refraktometer, ein Goniometer oder ein Michelson-Interferometer umfasst.

9. Messeinrichtung (100) nach Anspruch 6, 7 oder 8, wobei die Messeinrichtung (100) eine Erfassungseinrichtung zum Erfassen von Daten der Flüssigkeit in einem Tank (105), insbesondere Gewicht oder Volumen, umfasst.

10. Messeinrichtung (100) nach Anspruch 6, 7, 8 oder 9, wobei die Messeinrichtung eine Tankuhr zum Anzeigen der gespeicherten oder speicherbaren Energiemenge in dem Tank umfasst.

11. Messeinrichtung (100) nach Anspruch 6, 7, 8, 9, oder 10, wobei die Erfassungseinrichtung (101) ein Aräometer, ein Pyknometer, eine hydrostatische Waage oder eine Einrichtung zur Schwingungsmessung umfasst.

12. Fahrzeug mit einem Tank (107) für eine Flüssigkeit, die einen oder mehrere hydrierbare flüssige Wasserstoffträger umfasst, mit der Messeinrichtung (100) nach einem der Ansprüche 6 bis 11.

## Claims

1. Method for detecting a degree of hydrogenation of a liquid (107) which comprises one or more liquid hydrogen carriers which can be hydrogenated, having the following steps:
detecting (S101) a material property of the liquid (107); and
determining (S102) the degree of hydrogenation of the liquid (107) on the basis of the detected material property of the liquid (107),
**characterized in that** the degree of hydrogenation of the liquid (107) is determined on the basis of a linear relationship of the degree of hydrogenation and the detected material property, and wherein the liquid (107) comprises a mixture of dibenzyl toluene and at least partially hydrogenated dibenzyl toluene, or a mixture of benzyl toluene and at least partially hydrogenated benzyl toluene, or a mixture of benzyl toluene, dibenzyl toluene, at least partially hydrogenated dibenzyl toluene, and at least partially hydrogenated benzyl toluene.

2. Method according to Claim 1, wherein in the step of detecting (S101) the material property, at least one of the following material properties is detected: density, optical index of refraction, relative permittivity, speed of sound, viscosity, adsorption, absorption, or at least one material property which is derivable from material data such as density of the liquid (107).

3. Method according to Claim 1 or 2, wherein the material property is an optical index of refraction, and wherein the optical index of refraction is determined by means of a refractometer, a goniometer, or a Michelson interferometer.

4. Method according to any one of the preceding claims, furthermore having the steps of detecting the density of the liquid (107) and determining the degree of hydrogenation based on the density of the liquid (107).

5. Method according to any one of the preceding claims, wherein the material property, in particular density, is determined by means of a hydrometer, a pycnometer, a hydrostatic weighing, or an oscillation measurement.

6. Measuring device (100) for detecting a degree of hydrogenation of a liquid (107), which comprises one or more liquid hydrogen carriers which can be hydrogenated, having:
a detection device (101) for detecting a material property of the liquid (107); and
a determination device (103) for determining the degree of hydrogenation on the basis of the detected material property of the liquid (107),
wherein the degree of hydrogenation of the liquid (107) is determined on the basis of a linear relationship of the degree of hydrogenation and the detected material property,
**characterized in that** the measuring device (100) comprises a calculation device set up for calculating a stored or storable quantity of energy in the tank (105) on the basis of the degree of hydrogenation and the quantity of liquid (107),
wherein the liquid (107) comprises a mixture of dibenzyl toluene and at least partially hydrogenated dibenzyl toluene, or a mixture of benzyl toluene and at least partially hydrogenated benzyl toluene, or a mixture of benzyl toluene, dibenzyl toluene, at least partially hydrogenated dibenzyl toluene, and at least partially hydrogenated benzyl toluene.

7. Measuring device (100) according to Claim 6, wherein the detection device (101) is configured to detect at least one of the following material properties: density, optical index of refraction, relative permittivity, speed of sound, viscosity, adsorption, absorption, or at least one material property which is derivable from material data such as density of the liquid (107).

8. Measuring device (100) according to Claims 6 or 7, wherein the material property is an optical index of refraction, and wherein the detection device (101) comprises a refractometer, a goniometer, or a Michelson interferometer.

9. Measuring device (100) according to Claims 6, 7 or 8, wherein the measuring device (100) comprises a detection device for detecting data of the liquid in a tank (105), in particular weight or volume.

10. Measuring device (100) according to Claims 6, 7, 8 or 9, wherein the measuring device comprises a fuel gauge for displaying the stored or storable quantity of energy in the tank.

11. Measuring device (100) according to Claims 6, 7, 8, 9 or 10, wherein the detection device (101) comprises a hydrometer, a pycnometer, a hydrostatic scale, or a device for oscillation measurement.

12. Vehicle having a tank (107) for a liquid, which comprises one or more liquid hydrogen carriers which can be hydrogenated, having the measuring device (100) according to any one of Claims 6 to 11.

## Revendications

1. Procédé d'acquisition d'un degré d'hydrogénation d'un liquide (107), qui comprend un ou plusieurs porteurs d'hydrogène liquides hydrogénables, comprenant les étapes suivantes :
l'acquisition (S101) d'une propriété de matière du liquide (107) ; et
la détermination (S102) du degré d'hydrogénation du liquide (107) à partir de la propriété de matière acquise du liquide (107),
**caractérisé en ce que** le degré d'hydrogénation du liquide (107) est déterminé à partir d'une relation linéaire entre le degré d'hydrogénation et la propriété de matière acquise,
le liquide (107) comprenant un mélange de dibenzyltoluène et de dibenzyltoluène au moins partiellement hydrogéné, ou un mélange de benzyltoluène et de benzyltoluène au moins partiellement hydrogéné, ou un mélange de benzyltoluène, de dibenzyltoluène, de dibenzyltoluène au moins partiellement hydrogéné et de benzyltoluène au moins partiellement hydrogéné.

2. Procédé selon la revendication 1, dans lequel, lors de l'étape d'acquisition (S101) de la propriété de matière, au moins une des propriétés de matière suivantes est acquise : la densité, l'indice de réfraction optique, la permittivité relative, la vitesse du son, la viscosité, l'adsorption, l'absorption ou au moins une propriété de matière qui peut être dérivée de données de matière telles que la densité du liquide (107).

3. Procédé selon la revendication 1 ou 2, dans lequel la propriété de matière est un indice de réfraction optique, et dans lequel l'indice de réfraction optique est déterminé au moyen d'un réfractomètre, d'un goniomètre ou d'un interféromètre de Michelson.

4. Procédé selon l'une quelconque des revendications précédentes, comprenant en outre les étapes d'acquisition de la densité du liquide (107) et de détermination du degré d'hydrogénation à partir de la densité du liquide (107).

5. Procédé selon l'une quelconque des revendications précédentes, dans lequel la propriété de matière, notamment la densité, est déterminée au moyen d'un aéromètre, d'un pycnomètre, d'une balance hydrostatique ou d'une mesure de vibration.

6. Dispositif de mesure (100) pour l'acquisition d'un degré d'hydrogénation d'un liquide (107) qui comprend un ou plusieurs porteurs d'hydrogène liquides hydrogénables, comprenant :
un dispositif d'acquisition (101) pour l'acquisition d'une propriété de matière du liquide (107) ;
et
un dispositif de détermination (103) pour la détermination du degré d'hydrogénation à partir de la propriété de matière acquise du liquide (107),
le degré d'hydrogénation du liquide (107) étant déterminé à partir d'une relation linéaire entre le degré d'hydrogénation et la propriété de matière acquise,
**caractérisé en ce que** le dispositif de mesure (100) comprend un dispositif de calcul conçu pour le calcul d'une quantité d'énergie stockée ou pouvant être stockée dans le réservoir (105) à partir du degré d'hydrogénation et de la quantité du liquide (107), le liquide (107) comprenant un mélange de dibenzyltoluène et de dibenzyltoluène au moins partiellement hydrogéné, ou un mélange de benzyltoluène et de benzyltoluène au moins partiellement hydrogéné, ou un mélange de benzyltoluène, de dibenzyltoluène, de dibenzyltoluène au moins partiellement hydrogéné et de benzyltoluène au moins partiellement hydrogéné.

7. Dispositif de mesure (100) selon la revendication 6, dans lequel le dispositif d'acquisition (101) est configuré pour acquérir au moins une des propriétés de matière suivantes : la densité, l'indice de réfraction optique, la permittivité relative, la vitesse du son, la viscosité, l'adsorption, l'absorption ou au moins une propriété de matière qui peut être dérivée de données de matière telles que la densité du liquide (107) .

8. Dispositif de mesure (100) selon la revendication 6 ou 7, dans lequel la propriété de matière est un indice de réfraction optique, et dans lequel le dispositif d'acquisition (101) comprend un réfractomètre, un goniomètre ou un interféromètre de Michelson.

9. Dispositif de mesure (100) selon la revendication 6, 7 ou 8, dans lequel le dispositif de mesure (100) comprend un dispositif d'acquisition pour l'acquisition de données du liquide dans un réservoir (105), notamment le poids ou le volume.

10. Dispositif de mesure (100) selon la revendication 6, 7, 8 ou 9, dans lequel le dispositif de mesure comprend un indicateur destiné à indiquer la quantité d'énergie stockée ou pouvant être stockée dans le réservoir.

11. Dispositif de mesure (100) selon la revendication 6, 7, 8, 9 ou 10, dans lequel le dispositif d'acquisition (101) comprend un aéromètre, un pycnomètre, une balance hydrostatique ou un dispositif pour la mesure de vibration.

12. Véhicule comprenant un réservoir (107) pour un liquide qui comprend un ou plusieurs porteurs d'hydrogène liquides hydrogénables, comprenant le dispositif de mesure (100) selon l'une quelconque des revendications 6 à 11.
